# FASCICULE DE BREVET EUROPEEN

(11) **EP 0 300 879 B1**
(45) Date de publication et mention de la délivrance du brevet: **14.03.2001**
(21) Numéro de dépôt: 88401815.1
(22) Date de dépôt: 12.07.1988
(51) Int. Cl.: C07D 209/48, C07D 209/76, C08K 5/34

(54) **Procédé de préparation d'imides halogénés et leur application comme agents d'ignifugation**
Verfahren zur Herstellung von Halogenid-Imiden und ihre Verwendung als Feuerfestmachende Mittel
Process for the preparation of halogenated imides and their use as a fire retardant additive

(30) Priorité: 24.07.1987 FR 8710586
(43) Date de publication de la demande: 25.01.1989
(73) Titulaire: Atofina, 92800 Puteaux (FR)
(72) Inventeur: Bonnet, Evelyne, F-60260 Lamorlaye (FR); Gurtner, Bernard, F-38000 Grenoble (FR)

(56) Documents cités:
- EP-A- 0 101 785
- EP-A- 0 238 374
- FR-A- 2 359 128
- FR-A- 2 369 261
- US-A- 3 734 925
- US-A- 3 873 567
- JOURNAL OF THE CHEMICAL SOCIETY, 1937, partie I, pages 16-36, The Chemical Society, Londres, GB; H.D.K. DREW et al.: "Chemiluminescent organic compounds. Part I. Isomeric simple and complex hydrazides of phthalic acid and mode of formation of phthalazine and isoindole rings"
- JOURNAL OF POLYMER SCIENCES, no. 22, partie C, 1969, pages 773-784; J. IDRIS JONES et al.: "The reaction of hydrazine with polyimides and its utility"
- I & EC PRODUCT RESEARCH AND DEVELOPMENT, vol. 8, no. 4, décembre 1969, pages 397-398; S.M. SPATZ et al.: "Some N-substituted tetrabromophthalimide fire-retardant additives"

## Description

La présente invention concerne la synthèse de polyhalogénoimides et, plus particulièrement, celle d'imides dérivés d'acides dicarboxyliques halogénés comme l'acide tétrabromophtalique.

Les polyhalogénoimides, notamment les tétrabromophtalimides et bis(tétrabromophtalimides), sont des composés bien connus qui trouvent une application comme retardateurs de flamme dans de nombreuses matières inflammables, en particulier dans les matières plastiques (voir, par exemple, l'article de S.M. SPATZ et al intitulé "Some N-substituted tetrabromophtalimide fire-retardant additives" dans Industrial and Engineering Chemistry Product Research and Development, vol. 8, n° 4 (1969) pages 397-398, ainsi que les brevets US 3 873 567 et FR 2 369 261 et les demandes JP 74-045062 et 75-064337).

Cependant, les procédés de préparation décrits dans les références précitées donnent des rendements médiocres et fournissent des produits qui contiennent très souvent des matières volatiles aux températures de mise en oeuvre dans certains matériaux polymériques et entraînent la corrosion des moules. En outre, l'emploi pour leur préparation de solvants organiques (notamment xylène, toluène, alcool, acide acétique), sélectionnés le plus souvent en raison de leur aptitude à former avec l'eau des mélanges azéotropiques permettant d'entraîner l'eau de condensation produite par la réaction d'imidification ou à dissoudre l'anhydride d'acide dicarboxylique halogéné, nécessite des opérations coûteuses de séparation et de récupération de ces solvants, ainsi que des moyens de séchage adaptés à l'élimination des vapeurs de solvants organiques.

Les inconvénients susmentionnés se rencontrent notamment dans le cas des polyhalogénoimides dérivés d'hydrazine et d'anhydrides d'acides dicarboxyliques halogénés. Il a maintenant été trouvé qu'il n'est pas indispensable d'utiliser un solvant organique pour dissoudre l'anhydride et/ou éliminer l'eau de condensation et qu'en opérant en milieu aqueux sous certaines conditions, on peut obtenir avec des rendements très élevés et sans problèmes de rejet d'effluents et d'environnement des produits qui sans purification préalable, conviennent parfaitement à l'ignifugation des matières macromoléculaires, y compris celles dont la mise en oeuvre s'effectue à haute température, particulièrement au-dessus de 250°C.

Le procédé selon l'invention qui consiste à condenser l'hydrazine avec un anhydride d'acide dicarboxylique halogéné, est caractérisé en ce que l'on effectue la réaction en milieu aqueux, ne contenant pas de solvant organique, à une température allant de 40 à 225°C, avec un rapport molaire anhydride/hydrazine inférieur à 2, en introduisant progressivement l'hydrazin dans la solution ou la dispersion d'anhydrine halogène préalablement chauffée.

Selon la présente invention on utilise :
- les anhydrides d'acides dicarboxyliques aromatiques (benzène, naphtalène, anthracène), de formule générale: 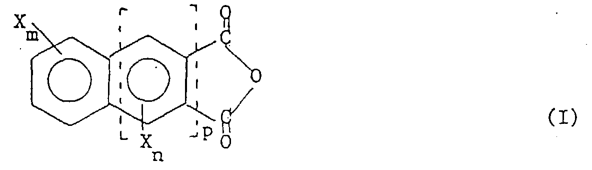 dans laquelle X représente un atome de brome ou de chlore, m est un nombre entier allant de 2 à 4, n et p sont des nombres entiers allant de 0 à 2 ;

- les anhydrides d'acides dicarboxyliques cycloaliphatiques, tels que l'acide hexabromo-1,4,5,6,7,7 bicyclo 2.2.1 heptène-5 dicarboxylique-2,3 et son homologue chloré (acide chlorendique), correspondant à la formule : 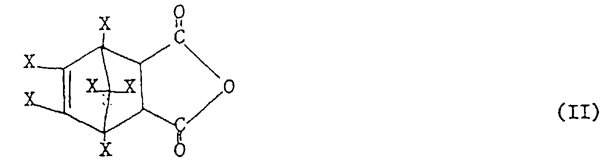 dans laquelle X a la même signification que ci-dessus. On utilise de préférence les anhydrides bromophtaliques, par exemple l'anhydride dibromo-3,4 (ou 3,6 ou 4,5) phtalique, l'anhydride tribromo-3,4,6 phtalique et plus particulièrement l'anhydride tétrabromophtalique.

Conformément à la présente invention, on peut utiliser un seul anhydride ou un mélange de deux ou plusieurs anhydrides. Les anhydrides commerciaux contenant, souvent une petite quantité d'acides minéraux peuvent être utilisés tels quels sans purification préalable. Il a en effet été constaté qu'on obtient des résultats particulièrement bons lorsque la solution ou dispersion aqueuse d'anhydride mise en oeuvre est acide ou fortement acide (pH inférieur à 7).

La quantité d'eau constituant le milieu réactionnel du procédé selon l'invention peut varier dans de larges limites, la seule condition étant qu'elle soit suffisante pour assurer la dispersion convenable des réactifs et permettre une bonne agitation. Cette condition est généralement réalisée en utilisant une quantité d'eau telle que la teneur en matières solides du milieu réactionnel soit comprise entre environ 5 et 75 % en poids', de préférence entre 20 et 40 %.

La réaction peut être effectuée sous pression atmosphérique à une température allant de 40 à 100°C ou, à condition d'opérer sous pression, à une température plus élevée pouvant aller jusqu'à 225°C. De préférence, on opère entre 100 et 225°C ce qui correspond à des pressions comprises entre 1 et 25 bars environ.

L'hydrazine sous forme d'hydrate ou de sel d'hydrazinium (par exemple sulfate, hydrohalogénure, acétate) peut être utilisée telle quelle ou sous forme de solution aqueuse diluée. De préférence, on utilise l'hydrate d'hydrazine sous forme de solution aqueuse commerciale. Le procédé selon l'invention est mis en oeuvre en introduisant progressivement l'hydrazine dans la solution ou dispersion d'anhydride halogéné préalablement chauffée et maintenue sous agitation.

La durée de réaction peut varier dans de larges limites, mais est généralement comprise entre 1 et 20 heures. Après refroidissement du milieu réactionnel, la suspension solide obtenue est filtrée et éventuellement lavée à l'eau jusqu'à neutralité, puis on séche le produit par les moyens de séchage classiques.

Le rapport molaire anhydride/hydrazine peut prendre différentes valeurs. Si on utilise un rapport voisin de 2 et une température au moins égale à 140°C, le produit obtenu est généralement constitué par le bis-imide répondant à la structure générale suivante: 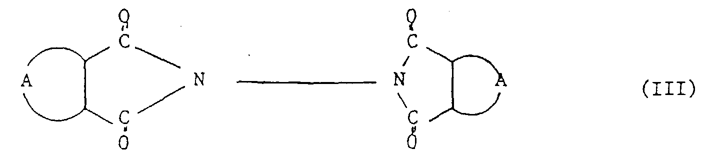 dans laquelle A représente le reste de l'anhydride halogéné mis en oeuvre.

Si on utilise un rapport inférieur à 1 ou voisin de 1, on obtient essentiellement le N-amino-imide de structure générale (IV) 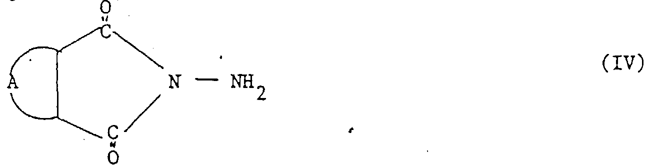 Lorsque le produit recherché est le N-amino-imide on utilise une quantité d'hydrazine comprise entre la stoechiométrie et 50 Z d'excès molaire et de préférence un rapport anhydride/hydrazine sensiblement égal à 1.

Si on utilise un rapport molaire anhydride/hydrazine compris entre 1 et 2 on obtient un mélange des produits précédents contenant d'autant plus de bis-imide que le rapport est plus près de 2 et que la température est plus élevée.

Qu'il s'agisse de bis-imides purs, de mélanges bis-imide + N-amino-imide + anhydride, ou de N-amino-amide, les produits obtenus conformément au procédé selon l'invention conviennent particulièrement bien comme retardateurs de flamme dans les matières plastiques de toute nature. Leur incorporation dans ces matières peut être effectuée par toute méthode connue à des doses allant de 5 à 40 % par rapport au poids de matière inflammable.

Les exemples suivants, dans lesquels les parties et les pourcentages sont exprimés en poids, illustrent l'invention sans la limiter.

Les produits ont été identifiés par analyse CHN et IR.

Le rendement est exprimé par rapport à l'anhydride.

### EXEMPLE 1

Dans un réacteur en verre, muni d'un agitateur et d'un dispositif de reflux, on disperse dans 2000 parties d'eau distillée 429 parties d'anhydride tétrachlorophtalique commercial. Le pH de la suspension est de 1,5.

Aprés avoir porté cette suspension à 60°C, on ajoute progressivement en 20 à 30 minutes 75 parties d'hydrate d'hydrazine 100 %, puis on porte le mélange réactionnel à 100°C. On ajoute ensuite 1000 parties d'eau distillée et maintient à 100°C pendant encore 10 heures. On obtient alors une suspension qu'on refroidit et filtre sur büchner. Aprés lavage à l'eau jusqu'a pH neutre et séchage sous vide à 110-120°C, on obtient avec un rendement supérieur à 97 Z un produit blanc dont l'analyse élémentaire et IR confirme la structure :

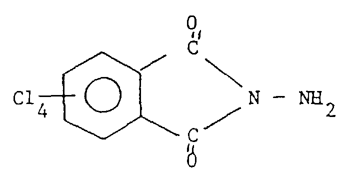

| Analyse élémentaire CHN | | |
|---|---|---|
| | trouvé | théorie |
| C % | 32,3 | 32,5 |
| H % | 0,7 | 0,7 |
| N % | 9,0 | 9,5 |

### EXEMPLE 2

On opère comme à l'exemple 1 en dispersant dans 4000 parties d'eau, 445 parties d'anhydride chlorendique commercial.

Le PH de la suspension est de 2,5.

On ajoute progressivement 60 g d'hydrate d'hydrazine 100 % dans la suspension portée à 98°C.

Après 10 heures de réaction à cette température on refroidit et on sépare le produit par filtration.

Après lavage jusqu'à PH neutre et séchage sous vide à 110°C-120°C, on obtient avec un rendement supérieur à 90 Z un produit gris dont l'analyse élémentaire et IR confirme la structure :

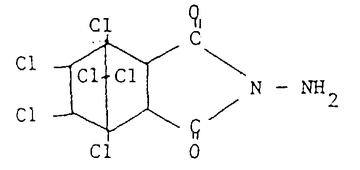

| Analyse élémentaire CHN | | |
|---|---|---|
| | Mesuré | théorique |
| C % | 27,9 | 28,1 |
| H % | 1,7 | 1,1 |
| N % | 7,3 | 7,3 |

### EXEMPLE 3

Dans un autoclave de six litres, muni d'un agitateur, on dispose 1392 parties d'anhydride tétrabromophtalique dans 4000 parties d'eau. Le PH de la dispersion est de 1.5.

On porte à 60°C puis on ajoute progressivement 225 parties d'hydrate d'hydrazine 100 %. On porte la suspension à 125°C sous pression et on maintient cette température pendant 10 heures.

Après ce temps de réaction on refroidit et on sépare le produit par filtration.

Après lavage jusqu'a PH neutre et séchage sous vide à 110-120°C, on obtient avec un rendement supérieur à 90 % un produit jaune dont l'analyse élémentaire et l'IR indique la structure :

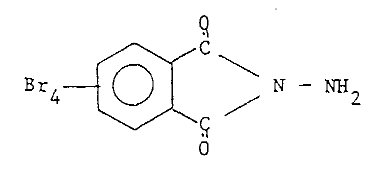

| Analyse CHN | | |
|---|---|---|
| | trouvé | théorique |
| % C | 19,58 | 20,1 |
| % H | 0,32 | 0,4 |
| % N | 5,68 | 5,9 |

### EXEMPLE 4

On opère comme dans l'exemple 3 mais en utilisant 100 % d'excès molaire d'hydrate d'hydrazine. On obtient le même produit que dans l'exemple 3 avec un rendement de 97,4 %.

### EXEMPLE 5

Dans un autoclave de quatres litres, muni d'un agitateur, on disperse 883 p. d'anhydride tétrabromophtalique commercial dans 2600 p. d'eau distillée ; on porte ce milieu, sous agitation, jusqu'à une température de 170°C, ce qui correspond à une pression entre 7,6 et 8 bars.

Au moyen d'une pompe, on introduit progressivement dans la suspension agitée 53 parties d'hydrate d'hydrazine, 100 % diluées avec 200 parties d'eau distillée (rapport molaire ATBP/HH = 1,8).

On maintient le milieu réactionnel durant 6 heures, toujours à 170°C, puis on refroidit, filtre et lave. Après séchage à 100-110°C sous vide on obtient avec un rendement de 95 % un produit blanc contenant :
83 % de N,N'-bis (tétrabromophtalimide)
14 % de N -aminotétrabromophtalimide
3 % d'anhydride tétrabromophtalique

### EXEMPLE 6

L'efficacité des produits selon l'invention comme agents d'ignifugation des matières plastiques a été testée suivant le mode opératoire général suivant :

A l'aide d'un mélangeur du type planétaire ou "tonneau", on mélange dans les proportions indiquées dans les tableaux suivants la résine sous forme de poudre ou de granulés avec l'ignifugeant à tester et les additifs éventuels (trioxyde d'antimoine, paraffine). Après homogénéisation, on extrude le mélange au moyen d'une extrudeuse appropriée (type double-vis, mono-vis ou "Buss") équipée ou non d'un moyen de dégazage, en opérant à la température d'extrusion indiquée par le fournisseur de résine et liée à la température de fusion du polymère. Le compound obtenu, éventuellement granulé, est ensuite transformé en éprouvettes de mesure au moyen d'une presse à injecter opérant aux températures convenables. Ces éprouvettes sont ensuite soumises aux tests normalisés de résistance au feu UL94 en 3,2 mm et 1,6 mm (Norme NF T51072) et d'indice d'oxygène I0 % (Norme NF T51071).

Le tableau A résume les résultats obtenus dans le cas du polybutylène téréphtalate (TMNO-ORGATER, granulés commercialisés par la Demanderesse).

| ESSAI N° | I témoin | II | III | IV |
|---|---|---|---|---|
| Ignifugeant de l'exemple n° | Néant | 1 | 3 | 5 |
| Composition (parties en poids) : | | | | |
| - Résine TMNO-ORGATER | 1000 | 1000 | 1000 | 1000 |
| - Ignifugeant | 0 | 142 | 160 | 186 |
| - Trioxyde d'antimoine | 0 | 74 | 76 | 78 |
| - Paraffine | 0 | 18 | 18 | 18 |

| Résultats des tests : | | | | |
|---|---|---|---|---|
| - UL94 | NC | VO | VO | VO |
| - I0 % | 22 | 27,6 | 32,2 | 31,3 |
| Couleur de l'éprouvette : | blanc | blanc ivoire | jaune | blanc ivoire |

## Revendications

1. Procédé de préparation d'imides halogénés par condensation d'hydrazine et d'anhydride d'acide dicarboxylique halogéné de formule : 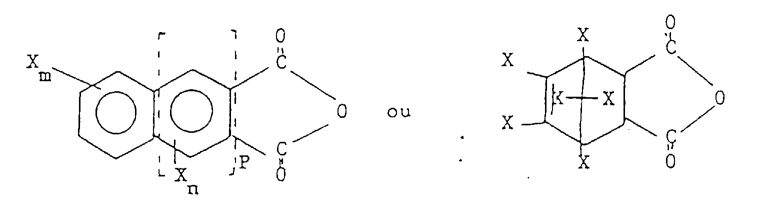 où X représente un atome de brome ou de chlore, m est un nombre entier allant de 2 à 4, n et p sont des nombres entiers allant de 0 à 2, ou un mélange de tels anhydrides,
caractérise en ce que la réaction est effectuée en milieu aqueux ne contenant pas de solvant organique, à une température allant de 40°C à 225°C, avec un rapport molaire anhydride/hydrazine inférieur à 2, en introduisant progressivement l'hydrazine dans la solution ou dispersion d'anhydride halogéné préalablement chauffée.

2. Procédé selon la revendication 1, dans lequel on opère sous pression à une température d'au moins 140°C.

3. Procédé selon la revendication 1 ou 2, dans lequel on part d'une solution ou dispersion aqueuse d'anhydride ayant un pH inférieur à 7.

4. Procédé selon la revendication 1, dans lequel on utilise l'anhydride tétrabromophtalique, l'anhydride tétrachlorophtalique, l'anhydride chlorendique, l'anhydride bromendique ou un mélange de ces anhydrides.

5. Procédé selon l'une des revendications 1 à 4, dans lequel l'hydrazine est utilisée sous forme d'hydrate ou de sel d'hydrazinium.

6. Procédé selon l'une des revendications 1 à 5 dans lequel on utilise un rapport molaire anhydride/hydrazine compris entre 1 et 2.

7. Procédé selon l'une des revendications 1 à 5 dans lequel on utilise un rapport molaire anhydride/hydrazine sensiblement égal à 1.

## Patentansprüche

1. Verfahren zur Herstellung von halogenierten Imiden durch Kondensation von Hydrazin und einem halogenierten Dicarbonsäureanhydrid der Formel: 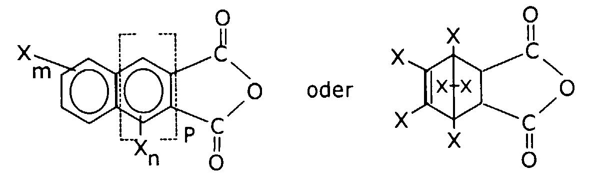 in der X ein Brom- oder Chloratom bedeutet, m eine ganze Zahl von 2 bis 4 ist, n und p ganze Zahlen von 0 bis 2 sind, oder einem Gemisch solcher Anhydride, dadurch gekennzeichnet, daß die Reaktion in einem wäßrigen Medium, das kein organisches Lösungsmittel enthält, bei einer Temperatur von 40 °C bis 225 °C mit einem Molverhältnis Anhydrid/Hydrazin kleiner 2 durchgeführt wird, wobei das Hydrazin nach und nach der zuvor erwärmten Lösung oder Dispersion des halogenierten Anhydrids zugegeben wird.

2. Verfahren nach Anspruch 1, in dem man unter Druck bei einer Temperatur von mindestens 140 °C arbeitet.

3. Verfahren nach Anspruch 1 oder 2, bei dem man von einer wäßrigen Lösung oder Dispersion des Anhydrids mit einem pH-Wert unter 7 ausgeht.

4. Verfahren nach Anspruch 1, bei dem man ein Tetrabromphthalsäure-, Tetrachlorphthalsäure-, Chlorendic- oder Bromendic-Anhydrid oder ein Gemisch dieser Anhydride verwendet.

5. Verfahren nach einem der Ansprüche 1 bis 4, bei dem das Hydrazin in Form eines Hydrats oder Hydraziniumsalzes verwendet wird.

6. Verfahren nach einem der Ansprüche 1 bis 5, bei dem man ein Molverhältnis Anhydrid/Hydrazin zwischen 1 und 2 verwendet.

7. Verfahren nach einem der Ansprüche 1 bis 5, bei dem man ein Molverhältnis Anhydrid/Hydrazin von ungefähr gleich 1 verwendet.

## Claims

1. Process for preparing halogenated imides by the condensation of hydrazine and a halogenated dicarboxylic acid anhydride of formula: 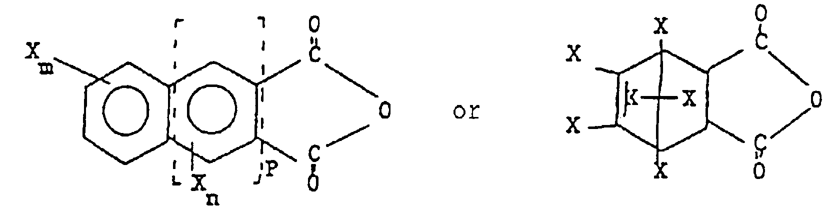 where X denotes a bromine or chlorine atom, m is an integer ranging from 2 to 4, and n and p are integers ranging from 0 to 2, or a mixture of such anhydrides, characterized in that the reaction is performed in aqueous medium containing no organic solvent, at a temperature ranging from 40°C to 225°C, with a mole ratio anhydride/hydrazine of less than 2, by gradually introducing the hydrazine into the pre-heated solution or dispersion of halogenated anhydride.

2. Process according to Claim 1, in which the reaction is performed under pressure at a temperature of at least 140°C.

3. Process according to Claim 1 or 2, in which the reaction is performed starting with an aqueous dispersion or solution of anhydride having a pH of less than 7.

4. Process according to Claim 1, in which tetrabromophthalic anhydride, tetrachlorophthalic anhydride, chlorendic anhydride or bromendic anhydride or a mixture of these anhydrides, is used.

5. Process according to one of Claims 1 to 4, in which hydrazine is used in the form of a hydrate or hydrazinium salt.

6. Process according to one of Claims 1 to 5, in which a mole ratio anhydride/hydrazine of between 1 and 2 is used.

7. Process according to one of Claims 1 to 5, in which a mole ratio anhydride/hydrazine substantially equal to 1 is used.
